# EUROPÄISCHE PATENTANMELDUNG

(11) **EP 2 345 886 A1**
(43) Veröffentlichungstag der Anmeldung: **20.07.2011**
(21) Anmeldenummer: 10000133.8
(22) Anmeldetag: 08.01.2010
(51) Int. Cl.: G01N 21/05, G01N 21/85, G01N 1/20, G01P 13/00, G01N 21/15

(54) **Prüfzelle und Fluidprüfsystem**

(71) Anmelder: Grundfos Management A/S, 8850 Bjerringbro (DK)
(72) Erfinder: Bentien, Anders, 8541 Skodstrup (DK)
(74) Vertreter: Hemmer, Arnd

(57) **Zusammenfassung**

Die Erfindung betrifft eine Prüfzelle zur visuellen Prüfung eines in einer Leitung (2) strömenden Fluids mit einen Prüfraum (12; 38), welcher einen Anschluss (8) zum Verbinden mit der Leitung (2) und in zumindest einer Außenwandung zumindest ein Sichtfenster (22) aufweist, sowie ein Fluidprüfsystem mit einer solchen Prüfzelle.

## Beschreibung

### Beschreibung

Die Erfindung betrifft eine Prüfzelle zur visuellen Prüfung eines in einer Leitung strömenden Fluids.

Beispielsweise in der Trinkwasserversorgung ist es erforderlich, die Wasserqualität kontinuierlich zu überwachen. Neben chemischer und biologischer Kontrollen kann hierzu eine visuelle Prüfung in Betracht kommen. Dazu ist es bekannt, Kameras einzusetzen, welche entsprechende Bildinformationen des Fluids aufnehmen, anhand derer dann die Qualität des Fluids bzw. des Wassers festgestellt wird. Dabei ist es jedoch schwierig eine Kamera in eine strömungsführende Leitung so zu integrieren, dass dort ein Bild des geförderten Fluids bzw. Wassers aufgenommen werden kann.

Es ist daher Aufgabe der Erfindung, eine Vorrichtung zu schaffen, welche auf einfache Weise eine visuelle Prüfung eines in einer Leitung strömenden Fluids ermöglicht.

Diese Aufgabe wird durch eine Prüfzelle mit den im Anspruch 1 angegebenen Merkmalen sowie durch ein Fluidprüfsystem mit den in Anspruch 15 angegebenen Merkmalen gelöst. Bevorzugte Ausführungsformen ergeben sich aus den Unteransprüchen, der Beschreibung sowie den Figuren.

Die erfindungsgemäße Prüfzelle dient zur visuellen Prüfung eines in einer Leitung strömenden Fluids. Dazu ist die Prüfzelle so angeordnet, dass sie an der Leitung, durch welche ein Fluid, beispielsweise Wasser, strömt, angebracht werden kann. Die Prüfzelle weist einen Prüfraum auf, in welchen das zu prüfende Fluid strömt und in welchem die visuelle Prüfung vorgenommen werden kann. Der Prüfraum weist dazu in zumindest einer Außenwandung zumindest ein Sichtfenster auf, durch welches hindurch eine visuelle Prüfung des Fluids im Inneren des Prüfraumes möglich ist. So kann beispielsweise außerhalb des Prüfraumes vor dem Sichtfenster eine Kamera platziert werden, um eine Beobachtung des Fluids im Inneren des Prüfraumes vorzunehmen. Der Prüfraum weist einen Anschluss auf, welcher zum Verbinden mit der Leitung, durch welche das Fluid strömt, vorgesehen ist. Durch diesen Anschluss wird Fluid aus der Leitung abgezweigt und in den Prüfraum geleitet. D. h. die erfindungsgemäße Prüfzelle ermöglicht die kontinuierliche Prüfung des Fluids, welches durch eine Leitung strömt, außerhalb der Leitung. Dadurch ist es nicht erforderlich, eine Kamera in die Leitung hinein zu bringen oder direkt an der Leitung anzuordnen. So kann beispielsweise eine Kamera nicht an der außerhalb der Leitung gelegenen Prüfzelle, d. h. am Sichtfenster des Prüfraumes, angebracht werden.

Der Prüfraum ist vorzugsweise geschlossen ausgebildet, kann jedoch zusätzlich eine Möglichkeit zur Probenentnahme aufweisen, beispielsweise mit einem Entnahmeanschluss oder -ventil versehen sein. Der Anschluss des Prüfraumes zu der Leitung hin ist vorzugsweise so ausgebildet, dass durch diesen ein kontinuierlicher Hin- und Rückfluss des durch die Leitung strömenden Fluids in den Prüfraum und wieder aus diesem hinaus zurück in die Leitung gewährleistet wird. Auf diese Weise kann ein stetiger Austausch des in dem Prüfraum vorhandenen Fluids gewährleistet werden, so dass stets die aktuelle Fluidqualität des Fluids, welches gerade durch die Leitung strömt, in der Prüfzelle bzw. im Prüfraum überwacht werden kann.

Der Anschluss an den Prüfraum ist vorzugsweise mit einem Gewinde zur Verbindung mit einem Anschlussstutzen einer Leitung versehen. Dieses

Gewinde kann ein Normgewinde sein, so dass die Prüfzelle mit diesem Gewinde an ihrem Anschluss an einen üblichen Anschlussstutzen einer Leitung, beispielsweise einem üblichen Entnahmeanschluss befestigt werden kann, ohne dass Modifikationen der Rohrleitung selber erforderlich sind. Auch ist hierüber ein leichter Austausch der Prüfzelle an der Leitung möglich. Der Anschlussstutzen an der Leitung ist vorzugsweise mit einem Absperrhahn versehen, so dass der Anschluss gesperrt werden kann, wenn die Prüfzelle beispielsweise abgenommen werden soll.

Weiter bevorzugt erstreckt sich durch den Anschluss hindurch zu dem Prüfraum hin ein Innenrohr, wobei sich das Innenrohr abgewandt von dem Prüfraum über den Anschluss derart hinaus erstreckt, dass es in eine mit dem Anschluss verbundenen Leitung hineinragen kann. Dies bedeutet, das Innenrohr ist an der dem Prüfraum abgewandten Seite so lang ausgebildet, dass es länger als ein an der Leitung vorgesehener Anschlussstutzen ist, so dass es sich durch einen solchen Anschlussstutzen hindurch in die Leitung hinein erstrecken kann, um dort einen Teil der Fluidströmung in die Prüfzelle hinein, d. h. in den Prüfraum hinein, abzweigen zu können. Am anderen Ende endet das Innenrohr an einer Eintrittsöffnung zu dem Prüfraum oder kann sich auch, je nach Ausgestaltung, weiter in den Prüfraum hinein erstrecken.

Bevorzugt dient das Innenrohr als Zufluss in den Prüfraum und ein das Innenrohr umgebender Freiraum und/oder ein zweiter Kanal in dem Innenrohr und/oder ein weiteres Innenrohr dient als Rückfluss von dem Prüfraum in die Leitung. So kann ein kontinuierlicher Fluidstrom durch den Prüfraum hindurch sichergestellt werden. Beispielsweise tritt das Fluid durch das Innenrohr in den Prüfraum ein und aus diesem durch einen das Innenrohr umgebenden Freiraum durch den Anschluss und gegebenenfalls einen Anschlussstutzen an der Leitung hindurch wieder zurück in die Leitung. Alternativ oder zusätzlich kann ein zweikanaliges Innenrohr oder ein zusätzliches Innenrohr vorgesehen sein, in welchem Zu- und Rückfluss vorgesehen sind. Beide Ausführungsformen haben den Vorteil, dass, um Hin- und Rückfluss zu dem Prüfraum zu gewährleisten, an einem Anschlussstutzen der Leitung keine spezielle Ausgestaltung vorgenommen werden muss. Diese liegt vielmehr allein in der Prüfzelle, welche mit einem solchen Anschlussstutzen verbindbar ist.

Weiter bevorzugt ist die Außenseite des Prüfraumes bzw. der Prüfzelle im Bereich des Sichtfensters zur Anbringung einer Kamera ausgebildet. So können hier geeignete Befestigungs- oder Anbringungsmittel, beispielsweise Gewindebohrungen, Bolzen, Klemmelemente, Bajonettverbindung oder ähnliches zur Anbringung einer Kamera vorgesehen sein. Bevorzugt ist hier eine standardisierte Schnittstelle vorgesehen, welche die Anbringung unterschiedlicher Kameras an der Prüfzelle ermöglich, ohne dass spezielle Anpassungen der einzelnen Kameras erforderlich sind. Es ist jedoch auch denkbar, eine Kamera nicht direkt an der Prüfzelle zu befestigen, sondern unabhängig von dieser zu befestigen, so dass die Kamera selber mechanisch nicht mit der Prüfzelle verbunden ist. Dies hat den Vorteil, dass Erschütterungen von der Leitung, welche beispielsweise durch der Fluidströmung verursacht sind, nicht auf die Kamera übertragen werden. Darüber hinaus ist so gegebenenfalls eine unabhängige Ausrichtung von Prüfraum mit Sichtfenster und Kamera relativ zu einander möglich.

Gemäß einer weiteren bevorzugten Ausführungsform weist die Prüfzelle Strömungserzeugungsmittel zum Erzeugung einer Fluidströmung in dem Prüfraum auf. Die Strömungserzeugungsmittel können beispielsweise ein oder mehrere Pumpen- oder Flügelräder sein, welche eine Fluidströmung innerhalb der Prüfzelle, insbesondere von der Leitung durch den Anschluss hindurch in den Prüfraum und wieder zurück in die Leitung, erzeugen. Gegebenenfalls kann hierzu ein separater Antriebsmotor für ein solches Pumpen- oder Flügelrad in oder an der Prüfzelle angeordnet sein, insbesondere ein elektrischer Antriebsmotor. Durch diese Strömungserzeugungsmittel wird ein kontinuierlicher Fluidstrom durch die Prüfzelle und den Prüfraum hindurch erzeugt, so dass sichergestellt werden kann, dass stets ein Anteil des aktuell durch die Leitung strömenden Fluids in die Prüfzelle abgezweigt wird und dort aktuell zur visuellen Untersuchung bereit steht.

Weiter bevorzugt kann dazu ein über den Anschluss gebildeter Ein- und/oder Auslass zu dem Prüfraum derart ausgestaltet sein, dass in dem Prüfraum eine Fluidströmung aufgrund der Fluidströmung in der Leitung erzeugt wird. Dies kann insbesondere durch entsprechende Ausgestaltung eines Innenrohres, welches sich durch den Anschluss hindurch in die Leitung erstreckt, erfolgen. Beispielsweise kann das Innenrohr an seinem zur Anordnung in der Leitung vorgesehen Ende eine Eintrittsöffnung aufweisen, welche gegen die Strömungsrichtung in der Leitung gerichtet ist. Eine solche Eintrittsöffnung ist somit vorzugsweise zumindest teilweise in Umfangsrichtung des Innenrohres gerichtet, so dass die Fluidströmung in der Leitung in die Öffnung des Innenrohres hinein gerichtet ist. Auf diese Weise wird automatisch ein Teil des Fluidstromes in das Innenrohr gelenkt. Entsprechend wird der Rückfluss aus der Prüfzelle bzw. dem Prüfraum bevorzugt so ausgebildet, dass auf ihn kein oder ein geringerer Staudruck der Fluidströmung in der Leitung lastet. Vorzugsweise ist hierzu die Austrittsöffnung in der Leitung in Strömungsrichtung gerichtet oder aber quer zur Strömungsrichtung gerichtet, so dass die Strömung in der Leitung nicht direkt auf diese Öffnung gerichtet ist.

Weiter bevorzugt sind Reinigungsmittel zum Reinigen des Sichtfensters vorgesehen. Hierbei kann es sich um mechanische Einrichtungen handeln, welche das Sichtfenster von Verunreinigungen reinigen, so dass stets ein freier Blick in das Innere des Prüfraumes gewährleistet ist. Insbesondere kann es sich bei diesen Reinigungsmitteln um Wischeinrichtungen handeln, welche mechanisch über die Innenfläche, d. h. die dem Prüfraum zugewandte Seite des Prüffensters, bewegt werden.

Gemäß einer besonderen Ausführungsform der Erfindung kann ein Strömungserzeugungsmittel gleichzeitig als Reinigungsmittel für das Sichtfenster fungieren. Beispielsweise kann ein Pumpen- oder Flügelrad so ausgebildet sein, dass es gleichzeitig die Innenfläche des Sichtfensters überstreicht. So können einzelne Kanten der Schaufeln eines solchen Rades als Wisch- bzw. Abstreifelemente ausgebildet sein, welche in Kontakt mit der Innenseite des Sichtfensters über diese geführt werden, so dass das Sichtfenster gereinigt wird. Auch ist es denkbar, die Fluidströmung in dem Prüfraum so zu führen, dass ein Teil der Fluidströmung, gegebenenfalls mit erhöhter Geschwindigkeit, über die Oberfläche des Sichtfensters geführt wird, um diese zu reinigen.

Bevorzugt jedoch weisen, wie vorangehend beschrieben, die Reinigungsmittel zumindest ein bewegliches Reinigungselement auf. Dabei handelt es sich weiter bevorzugt um ein rotierendes oder linear bewegliches Reinigungselement, welches über die Innenfläche des Sichtfensters streifen kann, um dieses zu reinigen. Ein solches Reinigungselement kann beispielsweise von Kanten der Schaufeln eines Pumpen- oder Flügelrades gebildet sein oder an einem solchen Pumpen- oder Flügelrad befestigt sein, so dass es bei dessen Drehung berührend über die Innenfläche des Sichtfensters geführt wird.

Die Strömungserzeugungsmittel und/oder die Reinigungsmittel im Inneren des Prüfraumes sind weiter bevorzugt über eine Magnetkupplung antreibbar. Dies hat den Vorteil, dass keine Wellen in den Prüfraum hinein geführt werden müssen. Daher werden auch keine problematischen Wellendichtungen erforderlich, welche gerade bei höheren Drücken innerhalb der Leitung eine Gefahr von Leckage darstellen könnten. Eine Magnetkupplung lässt die vollständig dichte Ausgestaltung des Prüfraums ohne Öffnungen in dessen Wandung zu. Hierzu rotiert ein erster Teil der Magnetkupplung außerhalb des Prüfraumes in Nähe dessen Wandung, während ein zweiter Teil der Magnetkupplung angetrieben im Inneren des Prüfraumes in Nähe dieser Wandung rotiert.

Gemäß einer besonderen Ausführungsform der Erfindung kann der Prüfraum relativ zu dem Anschluss beweglich sein. D. h. der Prüfraum ist dann, wenn der Anschluss an der Leitung befestigt ist, auch relativ zu der Leitung beweglich. Dies ermöglicht es zum einem, den Prüfraum in gewünschter Weise, beispielsweise relativ zu einer Kamera, auszurichten. Zum anderen hat diese bewegliche Anordnung auch den Vorteil, dass es möglich ist, den Prüfraum von der Leitung mechanisch in gewisser Weise zu entkuppeln, so dass Vibrationen, welche beispielsweise von der Strömung im Inneren der Leitung erzeugt werden, nicht oder nicht im vollen Umfang auf den Prüfraum übertragen werden. Hierdurch kann dann wiederum die visuelle Kontrolle verbessert werden, da ein ruhigeres Bild erzeugt werden kann. Ferner kann der Prüfraum über einen Motor, z. B. einen piezoelektrischen Motor, beweglich sein. Dieser kann z. B. Vibrationen auf den Prüfraum erzeugen, welche zur Reinigung des Sichtfensters dienen können.

Gemäß einer weiteren bevorzugten Ausführungsform der Erfindung ist ein Zwischenstück vorgesehen, welches mit einem ersten Ende zur Verbindung mit dem Anschluss und mit einem zweiten Ende zur Verbindung mit der Leitung oder einem Anschlussstutzen einer Leitung ausgebildet ist. Ein solches Zwischenstück kann als Adapter fungieren, beispielsweise um eine Prüfzelle mit standardisiertem Anschluss an unterschiedlichen Anschlussstutzen von Rohrleitungen zu befestigen. Dazu können dann verschiedene Zwischenstücke vorgesehen sein, welche alle an einem Ende eine Anschlusskonfiguration zur Verbindung mit dem Anschluss aufweisen und am anderen Ende entsprechend unterschiedlich ausgestaltete Anschlusskonfigurationen für die unterschiedlichen Anschlussstutzen oder Verbindungsstellen von Rohrleitungen aufweisen. Ferner ist es möglich, verschiedene Zwischenstücke mit unterschiedlichen Geometrien, beispielsweise Längen oder auch gewinkelte Zwischenstücke auszubilden, um die Anordnung des Prüfraumes an der Leitung an unterschiedliche Einbausituationen anpassen zu können. So lässt sich dann beispielsweise durch unterschiedlich gewinkelte Zwischenstücke die Blickrichtung des Sichtfensters ändern, beispielsweise in Abhängigkeit davon, wo der erforderliche Raum oder Zugang zur Anbringung einer Kamera vorhanden ist.

Bevorzugt ist das Sichtfenster parallel zu einer Mittelachse des Anschlusses ausgerichtet. Wenn der Anschluss mit einem sich normal zu der Leitung erstreckenden Anschlussstutzen verbunden wird, ist somit das Sichtfenster bei dieser Konfiguration normal zu der Leitung ausgerichtet, so dass die Blickrichtung auf das Sichtfenster parallel zur Längsachse der Leitung verläuft. Hierdurch wird eine raumsparende Anordnung der Prüfzelle seitlich der Leitung möglich, da sich eine Kamera dann ebenfalls parallel zur Leitung erstrecken kann. Allerdings kann eine normal zur Längsachse des Anschlusses und somit parallel zur Längsachse der Leitung ausgerichtete Lage des Sichtfensters den Vorteil haben, dass eine momentenfreie Anbringung der Prüfzelle an der Rohrleitung möglich ist, da so Prüfzelle und Kamera derart axial fluchtend an dem Anschlussstutzen einer Rohrleitung befestigt werden können, dass alle Gewichtskräfte in Richtung der Längsachse des Anschlussstutzens auf diesen wirken und auf die Verbindung an dem Anschlussstutzen somit keine Momente erzeugen.

In einer weiteren bevorzugten Ausführungsform kann der Prüfraum zwei Sichtfenster aufweisen, welche vorzugsweise an entgegengesetzten Seiten des Prüfraumes angeordnet sind. Dabei sind die beiden Sichtfenster weiter bevorzugt gegenüberliegend zueinander angeordnet, so dass es beispielsweise möglich ist, an eines der Sichtfenster eine Beleuchtungseinrichtung anzuordnen, während am anderem gegenüberliegenden Sichtfenster eine Kamera zur visuellen Kontrolle angeordnet wird. Somit würde das Fluid im Inneren des Prüfraumes von wird. Somit würde das Fluid im Inneren des Prüfraumes von dem von der Beleuchtungseinrichtung erzeugten Licht durchleuchtet und dieses Bild dann entsprechend beispielsweise von einer Kamera aufgenommen. Auch ermöglicht die Anordnung mehrerer Sichtfenster die Anordnung mehrerer Kamerasysteme, so dass beispielsweise verschiedene Kameras für unterschiedliche Untersuchungszwecke gleichzeitig an dem Prüfraum angebracht werden können. Auch ist es möglich Beleuchtungsmittel direkt im Prüfraum anzuordnen oder in eine Wandung des Prüfraumes zu integrieren.

Um den Prüfraum in gewünschter Weise relativ zu der Leitung platzieren zu können, kann neben der Verwendung eines Zwischenstückes, wie es vorangehend beschrieben wurde, auch der Anschluss auf unterschiedliche Weise, beispielsweise gewinkelt, insbesondere um 90° gewinkelt, ausgebildet sein, um eine Anordnung des Prüfraumes und insbesondere einer an diesem angebrachten Kamera in gewünschter Lage, z. B. parallel zu der Leitung zu ermöglichen. Auch könnte der Anschluss gegebenenfalls fest mit der Leitung verbunden, beispielsweise verschweißt sein.

Gegenstand der Erfindung ist neben der Prüfzelle ein Fluidprüfsystem, welches eine Prüfzelle gemäß der vorangehenden Beschreibung aufweist, wobei diese Prüfzelle mit einer eine Fluidströmung führenden Leitung verbunden ist. Die Funktionsweise dieser Prüfzelle in Zusammenwirkung mit der Leitung ergibt sich aus der vorangehenden Beschreibung.

Nachfolgend wir die Erfindung beispielhaft anhand der beigefügten Figuren beschrieben. In diesen zeigt:
- Fig. 1: eine Schnittansicht einer Leitung mit einer daran angeordneten erfindungsgemäßen Prüfzelle,
- Fig. 2: eine Schnittansicht einer Leitung mit einer darin angeordneten Prüfzelle gemäß einer zweiten Ausführungsform der Erfindung,
- Fig. 3a und 3b: schematisch die Anordnung eines Flügelrades in der Prüfzelle,
- Fig. 4: eine Schnittansicht einer Leitung mit einer darin angeordneten Prüfzelle gemäß einer weiteren Ausführungsform der Erfindung,
- Fig. 5: eine Schnittansicht einer Leitung mit einer darin angeordneten Prüfzelle gemäß einer weiteren Ausführungsform der Erfindung,
- Fig. 6: eine schematische, perspektivische Ansicht einer Leitung mit einer darin angeordneten Prüfzelle gemäß einer weiteren Ausführungsform der Erfindung und
- Fig. 7: schematisch in einer Schnittansicht den Aufbau eines Innenrohres mit zwei Kanälen.

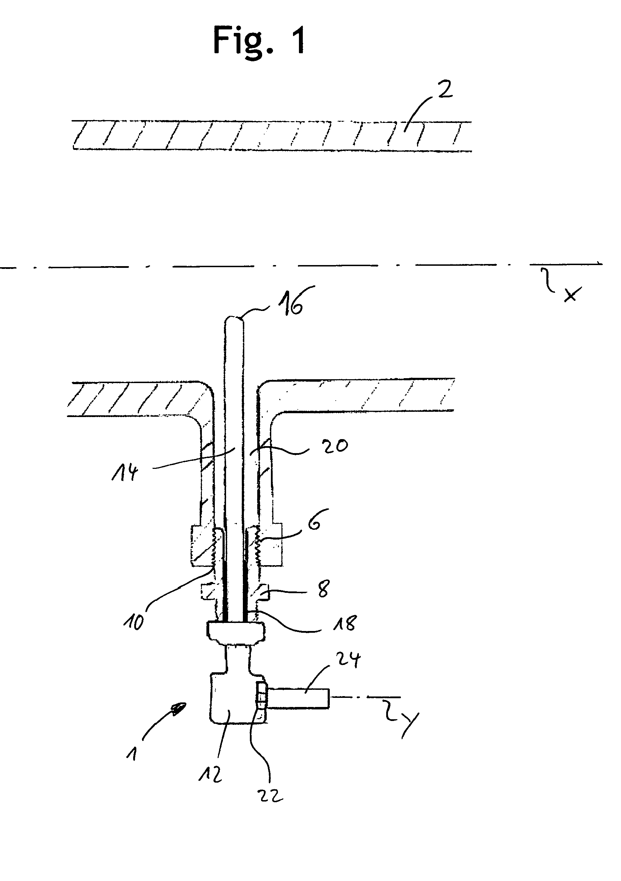

Fig. 1 zeigt ein erstes Ausführungsbeispiel einer erfindungsgemäßen Prüfzelle 1, welche an einer Leitung 2 angeordnet ist. Die Leitung 2 ist zum Führen einer Fluidströmung, beispielsweise einer Wasserströmung vorgesehen. Die Leitung 2 weist einen Anschlussstutzen 4 auf, welcher fest mit der Leitung 2 verbunden, beispielsweise verschweißt ist. Der Anschlussstutzen 4 erstreckt sich mit seiner Mittelachse normal bzw. radial zu der Längsachse X der Leitung 2. Dabei handelt es sich um einen üblichen Abzweig, welcher beispielsweise zum Anschluss abzweigender Leitungen oder auch eines Probenentnahmehahnes oder ähnlichem vorgesehen sein kann. An diesen Anschlussstutzen 4 ist die Prüfzelle 1 angesetzt. Der Anschlussstutzen 4 weist an seinem freien, der Leitung 2 abgewandten Ende ein Innengewinde 6 auf, in welches ein Anschluss 8 der Prüfzelle 1 eingeschraubt ist. Dazu weist der Anschluss 8 an seinem freien Ende ein zu dem Innengewinde 6 korrespondierendes Außengewinde 10 auf. Es ist zu verstehen, dass alternativ auch der Anschlussstutzen 4 ein Außengewinde und korrespondierend der Anschluss 8 ein Innengewinde aufweisen könnte.

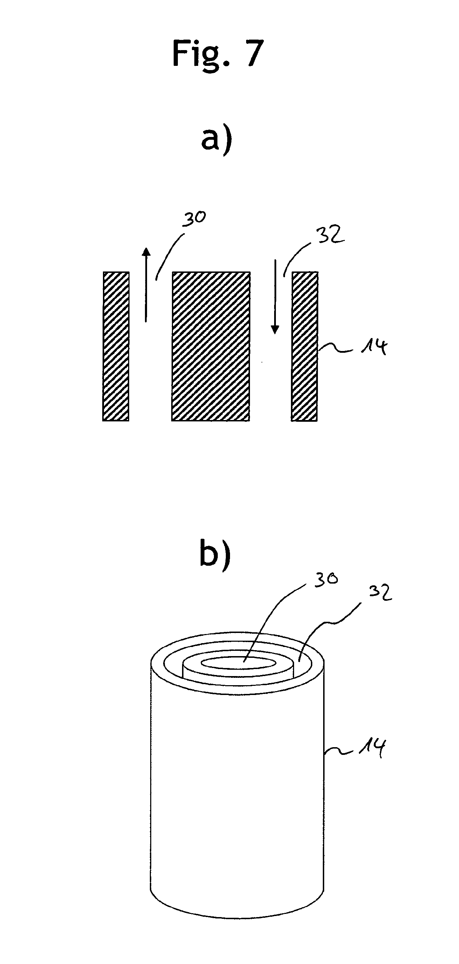

Der Anschluss 8 ist rohrförmig ausgestaltet und endet an seinem dem Gewinde 10 abgewandten Ende an einem Prüfraum 12 der Prüfzelle 1. Ausgehend von dem Prüfraum 12 erstreckt sich durch den Anschluss 8 hindurch ein Innenrohr 14. Das Innenrohr 14 ist dabei so lang ausgebildet, dass sich sein freies, dem Prüfraum 12 abgewandtes Ende über das der Leitung 2 zugewandte freie Ende des Anschlusses 8 so weit hinaus erstreckt, dass sich das Innenrohr 14 durch den Anschlussstutzen 4 bis in die Leitung 2 hinein erstreckt. Dabei ist das Innenrohr 14 an seinem in der Leitung 2 gelegenen Ende 16 mit zumindest einer Öffnung versehen. Das Innenrohr 14 bildet auf diese Weise einen Zufluss zu dem Prüfraum 12. D. h. das in der Leitung 2 strömende Fluid tritt durch eine Öffnung am Ende 16 des Innenrohres 14 in das Innenrohr 14 ein und fließt durch dieses in den Prüfraum 12. Der Rückfluss von dem Prüfraum 12 in die Leitung 2 erfolgt durch einen weiteren Kanal im Innenrohr 14, wie anhand von Fig. 7 erläutert wird. Im Inneren des Anschlusses 8 ist in dem das Innenrohr 14 umgebenden ringförmigen Spalt eine Dichtung 18 angeordnet, die den Spalt verschließt. Ohne diese Dichtung 18 könnte der Rückfluss auch durch den Freiraum 20 in dem Anschlussstutzen 4 erfolgen, der das Innenrohr 14 umgibt. Um den Eintritt der Strömung in das Innenrohr 14 am Ende 16 zu verbessern, kann eine dort gelegene Öffnung in Richtung der Längsachse X der Strömung entgegengerichtet sein, so dass das Fluid direkt in diese Öffnung eintritt.

Der Prüfraum 12 weist an einer Seitenwandung ein Sichtfenster 22 auf, durch welches ein Blick von außen in den ansonsten geschlossenen Prüfraum 12 möglich ist. Im hier gezeigten Beispiel ist außerhalb des Prüfraums 12 vor dem Sichtfenster 22 eine Kamera 24 zur Beobachtung des Fluids im Inneren des Prüfraumes 12 angeordnet. Bei dem hier gezeigten Ausführungsbeispiel ist das Sichtfenster 22 in einer Wandung des Prüfraumes 12 angeordnet, welche sich parallel zur Längsachse des Anschlussstutzen 4 und normal zur Längsachse X der Leitung 2 erstreckt. So ist die Blickrichtung durch das Sichtfenster 22 bzw. die Blickrichtung der Kamera 24 entlang einer Achse Y gerichtet, welches sich parallel zur Längsachse X der Leitung 2 erstreckt. Dies ermöglicht eine kompakte Anordnung des Kamerasystems seitlich zu der Leitung 2.

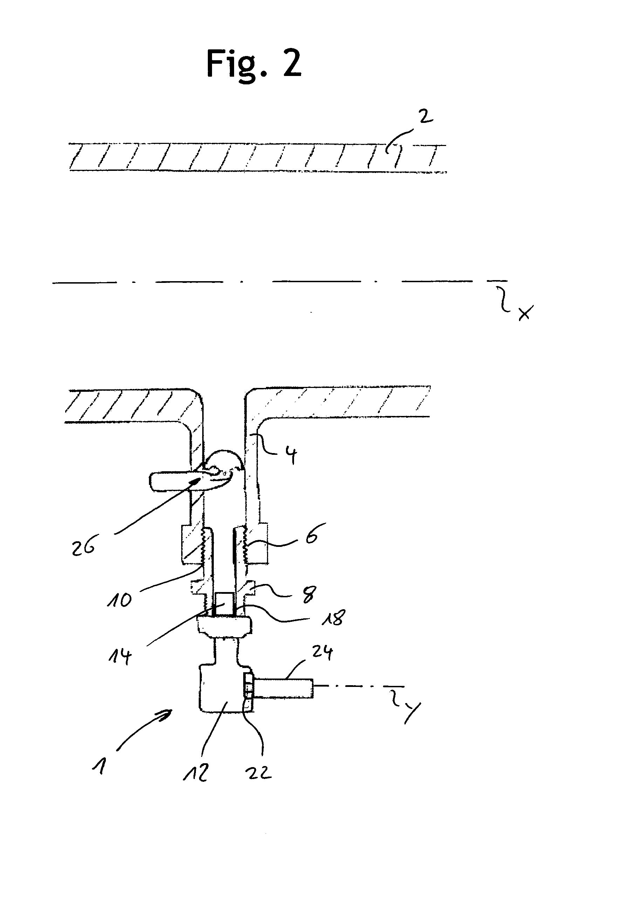

Bei der Ausführungsform gemäß Fig. 2 ist das Innenrohr 14 verkürzt, es endet innerhalb des Ausschlusses 8. Bei dieser Ausführungsform erfolgen Hin- und Rückfluss zu dem Prüfraum 12 somit durch einen gemeinsamen Kanal, welcher von dem Innenraum des Anschlussstutzen 4 und des Anschlusses 8 gebildet wird. Im Anschlussstutzen 4 ist zusätzlich ein Absperrhahn 26 angeordnet. Im Übrigen entspricht der Aufbau dem anhand von Fig. 1 beschriebene Aufbau.

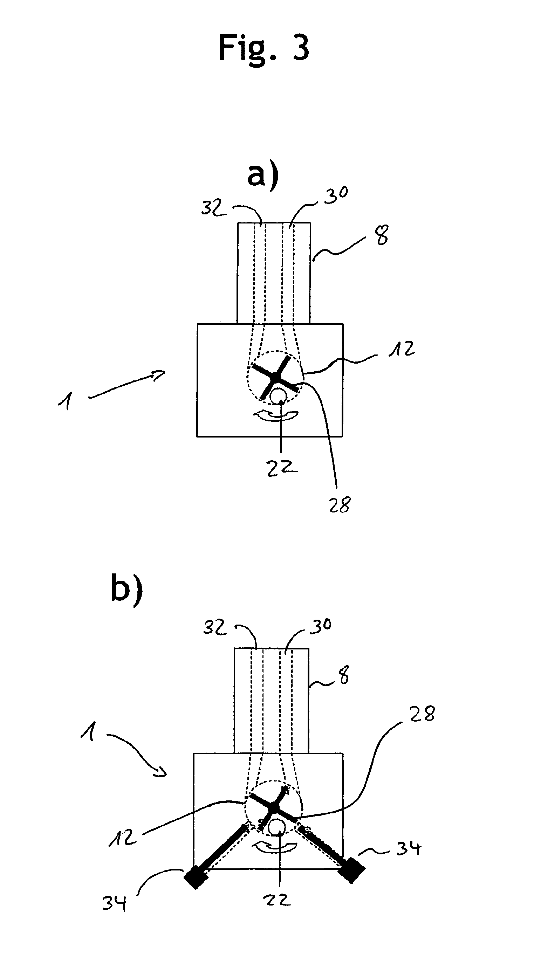

Um eine kontinuierliche Strömung durch den Prüfraum 12 und damit einen regelmäßigen Austausch des Fluids in den Prüfraum 12 zu gewährleisten, kann in der Prüfzelle 1 ein Strömungserzeugungsmittel vorgesehen sein. Ein Beispiel hierfür ist in den Figuren 3a und 3b gezeigt, welche schematisch eine Prüfzelle 1 zeigen. Bei dieser Ausführungsform ist in dem Prüfraum 12 der Prüfzelle 1 ein Flügelrad 28 angeordnet, welches bei Drehung in dem Prüfraum 12 eine Fluidströmung erzeugt. Für Hin- und Rückfluss dieser Fluidströmung von bzw. zu der Leitung 2 sind im Anschluss 8 zwei Strömungskanäle 30 und 32 ausgebildet. Diese können sich durch ein hier nicht gezeigtes Rohr bzw. Innenrohr (entsprechend Innenrohr 14 in Fig. 1) bis in die Leitung 2 hinein erstrecken. Im gezeigten Beispiel wird das Flügelrad 28 magnetisch beispielsweise über zwei gezielt bestrombare Elektromagneten 34 berührungslos drehend angetrieben. Das Flügelrad 28 ist dazu im Inneren des Prüfraumes 12 drehbar gelagert, ohne dass sich eine Antriebswelle aus dem Prüfraum 12 nach außen erstrecken müsste. Die Flügel des Flügelrades 28 sind magnetisiert oder mit Permanentmagneten versehen. Durch entsprechende Bestromung der Elektromagnete 34, welche außerhalb des Prüfraumes 12 angeordnet sind, können auf die Flügel des Flügelrades 28 bzw. die dort angeordneten Magnete wechselnde Anziehungs- und/oder Abstoßungskräfte ausgeübt werden, so dass das Flügelrad 28 in Rotation versetzt wird.

Das Sichtfenster 22 in dem Prüfraum 12 ist so angeordnet, dass es von den Flügeln des Flügelrades 28 überstrichen wird. Dabei können die sich normal zur Drehachse des Flügelrades erstreckenden Längskanten der Flügel, welche das Sichtfenster 22 überstreichen als Abstreifelemente bzw. Reinigungselemente ausgebildet sein, welche beim Überstreichen des Sichtfensters 22 dieses reinigen.

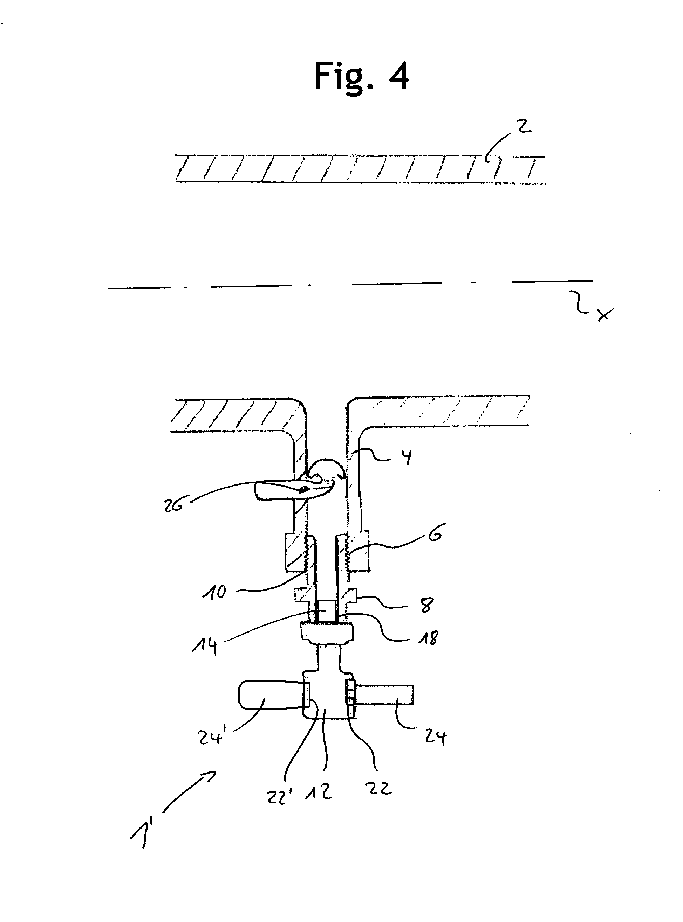

Fig. 4 zeigt eine Variante der Ausführungsform gemäß Fig. 2, bei welcher an dem Anschlussstutzen 4 eine Prüfzelle 1 mit zwei Kamaras 24 und 24' angeordnet ist

Dazu weist der Prüfraum 12 ein zweites Sichtfenster 22' an einer dem Sichtfenster 22 gegenüberliegenden Wandung auf. An dieses Sichtfenster ist die zweite Kamera 24' angesetzt. Die beiden Kameras 24 und 24' können beispielsweise unterschiedliche Beobachtungsaufgaben übernehmen. Bei der gegenüberliegenden Anordnung zweier Sichtfenster 22 und 22' wäre es darüber hinaus weiterhin möglich, nur eine Kamera 24 an dem Sichtfenster 22 vorzusehen und beispielsweise an dem Sichtfenster 22' eine Beleuchtungseinrichtung zur Durchleuchtung des Prüfraumes 12 anzuordnen. Zu- und Abfluss der Flüssigkeit von der Leitung 2 erfolgt wie bei dem Ausführungsbeispiel gemäß Figur 2 durch den Anschlussstutzen 4 und den Anschlussstutzen 8 in das Innenrohr 14 hinein in den Prüfraum 12 und durch denselben Weg wieder zurück.

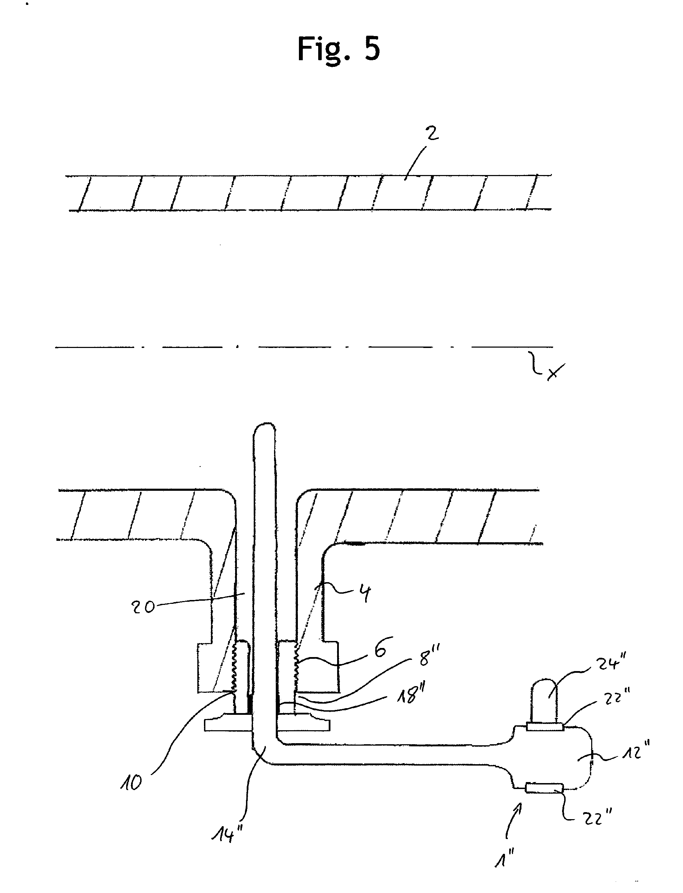

Figur 5 zeigt eine Variante der Ausführungsform gemäß Figur 1, bei welcher der Prüfraum 12 in einem Winkel von 90° verglichen mit dem Prüfraum 12 in Figur 1 angeordnet ist. Hierzu erstreckt sich das Innenrohr 14" axial aus dem Anschluss 8" heraus und ist dann um 90° gewinkelt und erstreckt sich dann parallel zu der Leitung 2 zu dem Prüfraum 12". D.h. hier ist der Prüfraum 12" nicht direkt an dem Anschluss 8" angeordnet, sondern über das Innenrohr 14" verbunden. Wie anhand von Figur 1 beschrieben, erfolgt Hin- und Rückfluss zu der Leitung 2 durch dieses Innenrohr 14", welches dazu zwei Innenkanäle aufweisen kann, wie anhand von Figur 7 beschrieben werden wird. In dem Anschluss 8" ist das Innenrohr 14" durch eine Dichtung 18" gedichtet eingesetzt.

Auch bei dieser Ausführungsform gemäß Figur 5 weist der Prüfraum 12" ähnlich der Ausführungsform gemäß Figur 4 zwei Sichtfenster 22" an zwei gegenüberliegenden Wandungen auf. An einem dieser Sichtfenster ist hier eine Kamera 24" angeordnet. Das andere Sichtfenster 22" ist frei und kann beispielsweise zur visuellen Prüfung mit dem Auge verwendet werden oder zur Beleuchtung des Inhaltes des Prüfraumes 12".

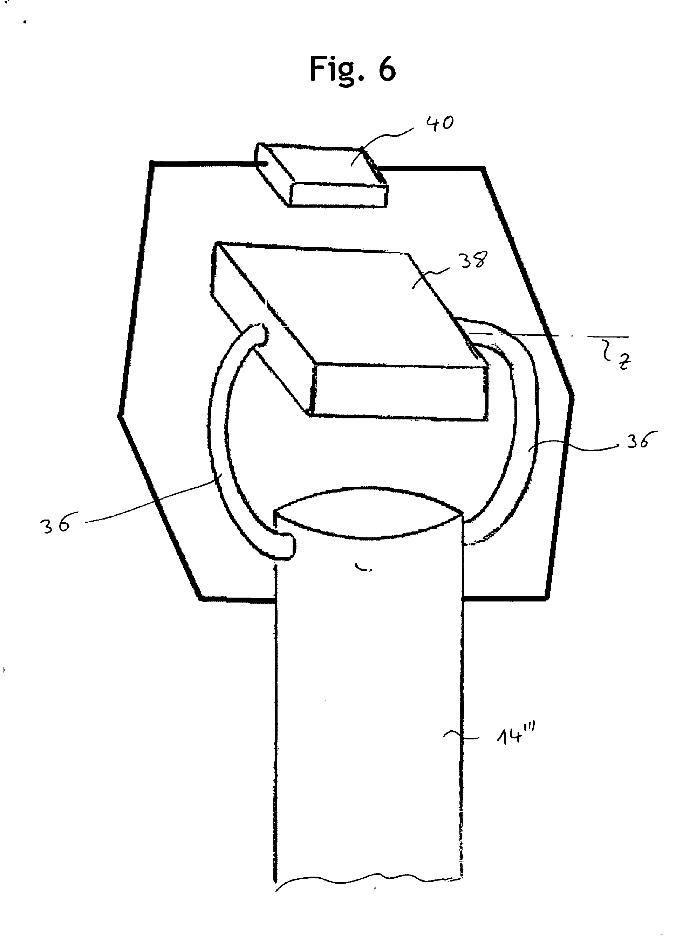

Fig. 6 zeigt schematisch eine weitere Ausführungsform der Erfindung. Bei dieser zweigen von einem Innenrohr 14"' zwei Anschlusskanäle 36 ab, welche zu dem Prüfraum 38 der Prüfzelle führen. Das Innenrohr 14"' kann sich beispielsweise in der in Figur 5 gezeigten Weise aus dem Anschluss 8 bzw. 8" heraus erstrecken, wobei es nicht wie in Figur 5 gezeigt gewinkelt ausgebildet sein muss. In Figur 6 ist nur das außerhalb des Anschlusses 8 angeordnete, der Leitung 2 abgewandte Ende des Innenrohres 14"' gezeigt. Von den Anschlusskanälen 36 dient einer dem Hin- und ein andere dem Rückfluss zu dem Prüfraum 38. Der Prüfraum 38 ist auch hier mit einem Sichtfenster versehen oder insgesamt transparent ausgebildet. Eine Kamera 40 ist gegenüberliegend zu dem Prüfraum 38 angeordnet. Dabei kann die Kamera 40 stationär getrennt von dem Prüfraum 38 befestigt werden, während der Prüfraum 38 beweglich ausgebildet sein kann, beispielsweise um eine Achse Z verschwenkbar sein kann, beispielsweise um ihn oder sein Sichtfenster relativ zu der stationären Kamera 40 auszurichten. Durch die Entkopplung von Kamera 40 und Prüfraum 38 wird ferner verhindert, dass Erschütterungen bzw. Vibrationen, welche von der Strömung im Inneren der Leitung 2 hervorgerufen werden, von der Prüfzelle bzw. dem Prüfraum 38 auf die Kamera 40 übertragen werden. So kann eine bessere Bildqualität erzeugt werden. Die Bewegung des Prüfraumes 38 kann beispielsweise zurück über einen elektrischen Motor erfolgen. Dieser kann auch dazu verwendet werden den Prüfraum 38 und/oder das Sichtfenster 40 derart in Vibrationen zu versetzen, dass seine Wandungen oder ggf. ein Sichtfenster beispielsweise durch Schwingungen im Ultraschallbereich gereinigt wird.

Fig. 7a zeigt schematisch den Schnitt durch ein Innenrohr 14 mit zwei Strömungskanälen 30 und 32, welche für Hin- und Rückfluss von der Leitung 2 zu dem Prüfraum 12 vorgesehen sind.

Figur 7b zeigt eine alternative Ausführungsform des Innenrohres, bei welcher die beiden Strömungskanäle 30 und 32 konzentrisch angeordnet sind. Hier kann beispielsweise der innere Strömungskanal 20 als Hinfluss und der äußere ringförmige Strömungskanal 32 als Rückfluss dienen. Auch eine umgekehrte Funktion ist denkbar.

### Bezugszeichenliste

- 1,1',1": - Prüfzelle
- 2: - Leitung
- 4: - Anschlussstutzen
- 6: - Innengewinde
- 8,8": - Anschluss
- 10: - Außengewinde
- 12, 12', 12": - Prüfraum
- 14, 14": - Innenrohr
- 16: - Ende
- 18, 18": - Dichtung
- 20: - Freiraum
- 22, 22', 22": - Sichtfenster
- 24, 24', 24": - Kamera
- 26: - Absperrhahn
- 28: - Flügelrad
- 30, 32: - Strömungskanäle
- 34: - Elektromagnete
- 36: - Anschlusskanäle
- 38: - Prüfraum
- 40: - Kamera
- X: - Längsachse der Leitung 2
- Y: - Blickrichtung der Kamera 24
- Z: - Schwenkachse

## Patentansprüche

1. Prüfzelle zur visuellen Prüfung eines in einer Leitung (2) strömenden Fluids, **gekennzeichnet durch** einen Prüfraum (12; 38), welcher einen Anschluss (8) zum Verbinden mit der Leitung (2) und in zumindest einer Außenwandung zumindest ein Sichtfenster (22) aufweist.

2. Prüfzelle nach Anspruch 1, **dadurch gekennzeichnet, dass** der Anschluss (8; 36) mit einem Gewinde (10) zur Verbindung mit einem Anschlussstutzen (4) einer Leitung (2) versehen ist.

3. Prüfzelle nach Anspruch 1 oder 2, **gekennzeichnet durch** ein sich **durch** den Anschluss (8; 36) zu dem Prüfraum (12; 38) erstreckendes Innenrohr (14), wobei sich das Innenrohr (14) abgewandt von dem Prüfraum (12; 38) über den Anschluss (8; 36) derart hinaus erstreckt, dass es in eine mit dem Anschluss (8; 36) verbundene Leitung (2) hineinragen kann.

4. Prüfzelle nach Anspruch 3, **dadurch gekennzeichnet, dass** das Innenrohr (14) als Zufluss in den Prüfraum(12; 38) und ein das Innenrohr umgebender Freiraum (18; 20) und/oder ein zweiter Kanal (30; 32) in dem Innenrohr (14) als Rückfluss von dem Prüfraum(12; 38) in die Leitung (2) vorgesehen ist.

5. Prüfzelle nach einem der vorangehenden Ansprüche, bei welcher die Außenseite des Prüfraums(12; 38) im Bereich des Sichtfensters (22) zur Anbringung einer Kamera (24; 40) ausgebildet ist.

6. Prüfzelle nach einem der vorangehenden Ansprüche, **gekennzeichnet durch** Strömungserzeugungsmittel, bevorzugt in Form eines Pumpen- oder Flügelrades (28), zum Erzeugen einer Fluidströmung in dem Prüfraum (12; 38).

7. Prüfzelle nach Anspruch 6, **dadurch gekennzeichnet, dass** ein über den Anschluss (8; 36) gebildeter Ein- und/oder Auslass zu dem Prüfraum (12; 38) derart ausgestaltet ist, dass in dem Prüfraum (12; 38) eine Fluidströmung aufgrund einer Fluidströmung in der Leitung (2) erzeugt wird.

8. Prüfzelle nach einem der vorangehenden Ansprüche, **gekennzeichnet durch** Reinigungsmittel zum Reinigen des Sichtfensters (22).

9. Prüfzelle nach Anspruch 8, **dadurch gekennzeichnet, dass** ein Strömungserzeugungsmittel (28) gleichzeitig als Reinigungsmittel für das Sichtfenster fungiert.

10. Prüfzelle nach Anspruch 8 oder 9, **dadurch gekennzeichnet, dass** die Reinigungsmittel zumindest ein bewegliches Reinigungselement, vorzugsweise zumindest ein rotierendes oder linear bewegliches Reinigungselement (18) aufweisen.

11. Prüfzelle nach einem der vorangehenden Ansprüche, bei welcher Strömungserzeugungsmittel und/oder Reinigungsmittel im Inneren des Prüfraumes (12; 38) über eine Magnetkupplung (34) antreibbar sind.

12. Prüfzelle nach einem der vorangehenden Ansprüche, **dadurch gekennzeichnet, dass** der Prüfraum (38) relativ zu dem Anschluss (36) beweglich ist.

13. Prüfzelle nach einem der vorangehenden Ansprüche **gekennzeichnet durch** ein Zwischenstück, welches mit einem ersten Ende zur Verbindung mit dem Anschluss (8; 36) und mit einem zweiten Ende zur Verbindung mit der Leitung (2) oder einem Anschlussstutzen (4) einer Leitung (2) ausgebildet ist.

14. Prüfzelle nach einem der vorangehenden Ansprüche, **dadurch gekennzeichnet, dass** sich das Sichtfenster (22) parallel zu einer Mittelachse des Anschlusses (8; 36) erstreckt.

15. Fluidprüfsystem **gekennzeichnet durch** eine Prüfzelle (1) gemäß einem der vorangehenden Ansprüche, welche mit einer eine Fluidströmung führenden Leitung (2) verbunden ist.
